# EUROPÄISCHE PATENTSCHRIFT

(11) **EP 0 468 351 B1**
(45) Veröffentlichungstag und Bekanntmachung des Hinweises auf die Patenterteilung: **11.10.1995**
(21) Anmeldenummer: 91111916.2
(22) Anmeldetag: 17.07.1991
(51) Int. Cl.: A61F 5/01

(54) **Orthopädische Therapie-Schuhe und -Innenschuhe**
Orthopaedic medical shoe and internal shoe
Chaussure interne et chaussure orthopédiques

(30) Priorität: 26.07.1990 DE 9011037 U
(43) Veröffentlichungstag der Anmeldung: 29.01.1992
(73) Patentinhaber: SOLOR SCHUHFORSCHUNG UND ENTWICKLUNG GmbH, D-66953 Pirmasens (DE)
(72) Erfinder: Birke, Josef, W-6780 Pirmasens (DE)
(74) Vertreter: Patentanwälte Möll und Bitterich

(56) Entgegenhaltungen:
- EP-A- 0 275 543
- DE-U- 8 809 794
- DE-U- 8 907 464
- DE-U- 9 004 108

## Beschreibung

Die Erfindung betrifft orthopädische Innenschuhe gemäß dem Oberbegriff des Anspruchs 1.

Die DE-U 89 07 464 zeigt eine Vorrichtung zum Stützen der Sprunggelenke, bestehend aus einem einstückig aus Polypropylen tiefgezogenem Paßformteil, das zwei Seitenteile, ein Fußbetteil, ein Rückenteil und ein über die ganze Länge offenes Vorderteil aufweist. Im Rückenteil ist eine Fersenaussparung vorgesehen. Weitere Aussparungen sind im Bereich der Knöchel angeordnet. Die beiden Seitenteile sind mit je einer Versteifungsrippe vorgesehen, die jeweils seitlich neben der Knöchelaussparung beginnt, von dort aus nach oben geführt wird, einen Bogen von annähernd 180 Grad beschreibt, dann wieder nach unten geführt ist und außenseitig auf der anderen Seite der Knöchelaussparung endet. Beide Seitenteile sind mit einem oder mehreren Schlitzen versehen, durch die Verschlußbänder, beispielsweise Klettverschlußbänder, gezogen werden.

Dank ihrer Herstellung aus thermoplastischem Kunststoff kann die bekannte Stützvorrichtung durch Erwärmen erweicht und so entsprechend dem Patientenfuß nachgeformt werden. Da die eigentliche Formgebung im Tiefziehverfahren erfolgt, wird als Ausgangsmaterial eine Kunststoffplatte einheitlicher Stärke verwendet, so daß auch die fertige Stützvorrichtung eine im wesentlichen überall gleichmäßige Stärke besitzt. Daraus resultiert zunächst ein relativ hohes Gewicht. Wesentlich unangenehmer ist jedoch, daß es an den steifen Rändern der Stützvorrichtung zu Druck- und Scheuerstellen kommt. Aus diesem Grunde wird vorgeschlagen, das Paßformteil auch innenseitig auszupolstern oder den Fuß des Patienten zusätzlich mit einer Gummitrikotbandage zu umwickeln. Diese Stützvorrichtungen besitzen daher nur eine geringe Akzeptanz bei den Patienten.

Eine weitere Sprunggelenkorthese ist bekannt aus der DE-U 90 04 108. Diese besteht aus einer einstückigen, vorn und hinten über die volle Höhe geschlitzten Schale aus einem wasserfesten Kunststoff. Zur Befestigung am Fuß dienen zwei Klettverschlußgurte, die an der Kunststoffschale angenietet sind und durch vorbereitete Schlitze hindurchgezogen werden können. Im Bereich der Knöchel können Aussparungen vorgesehen sein. Aufgrund der Verwendung einer Kunststoffplatte als Ausgangsmaterial zur Herstellung der Schalenform hat die fertige Orthese im wesentlichen überall eine gleichmäßige Dicke, so daß der Gefahr von Druck- und Scheuerstellen durch eine Polsterung vorgebeugt werden muß.

Neben den orthopädischen Innenschuhen der hier beschriebenen Art, die dazu bestimmt sind, das Sprunggelenk ruhig zu stellen, gibt es auch orthopädische Innenschuhe für Spastiker, da nach medizinischer Erkenntnis eine Entspannung des kindlichen Spastikerfußes und sogar eine Korrektur bis zur Normalstellung möglich ist, wenn der Fuß im rechten Winkel zum Unterschenkel fixiert wird. Die Therapie-Schuhe sollen jedoch nicht nur den Fuß ruhigstellen, sondern den Patienten auch das schmerzfreie Gehen mit einem normalen Gangbild ermöglichen.

Bei Lähmungen wird mit orthopädischen Innenschuhen in Form von sogenannten Lähmungskappen ein plantares Abkippen des Fußes verhindert. Das Gangbild wird normalisiert, indem der Auftritt von der Zehenspitze wieder auf die Ferse umgestellt wird. Die Stolper- und Sturzgefahr wird dadurch behoben, der Gang wieder normal, sicher und ermüdungsfrei.

Für alle diese Anwendungsfälle liefert die Industrie vorgeformte Platten aus thermoplastischem Kunststoff, der in vielen Fällen auch mit Verstärkungsfasern gefüllt ist. Daraus fertigt der Orthopädieschuhmacher die Arthrodesen-Kappen, Lähmungskappen, Knöchelkappen, indem er die Platten aussägt, erwärmt und über den Rückfuß des Patienten-Beinleistens preßt. Bei etwas Glück und viel Geschick hat das Kunststoffmaterial nach dem Erkalten die dem Bein und Rückfuß des Patienten entsprechende Form angenommen. Aus diesen rohen Formlingen müssen Endform und Endgröße sowie die richtigen Materialverteilungen noch herausgearbeitet werden. Hierzu wird das überschüssige Kunststoffmaterial mit Hilfe von Schleifscheiben und Raspeln abgearbeitet. Dieser Vorgang erfordert viel Erfahrung und Fingerspitzengefühl. Wird an der falschen Stelle zuviel Material abgenommen, war die ganze Arbeit umsonst; der Kunststoffkern muß neu hergestellt werden.

Es versteht sich, daß das Herausarbeiten der Endform aus dem Kunststoff-Rohling zeitaufwendig ist. Außerdem entsteht eine reichliche Menge an Kunststoff- und/oder Lederabfällen, zumeist in Staubform, deren Entsorgung immer schwieriger wird. Dies gilt insbesondere dann, wenn die Kunststoffe faserverstärkt sind. Auch führt der beim Abschleifen von Kunststoffen entstehende Staub oftmals zu Irritationen der Haut und der Atmungsorgane.

Schließlich liefert die Industrie auch grob vorgefertigte Bein- und Knöchelrohlinge aus Kunststoff. Auch diese müssen jedoch unter erheblichem Zeitaufwand individuell nachgearbeitet und insbesondere von Hand nachgeschmirgelt werden.

Falls man versuchen würde, die verformten Kunststoffplatten ohne weitere Nachbearbeitung zu verwenden, würde, bedingt durch die einheitliche Stärke des Plattenmaterials von 4 bis 6 mm, zu der sich noch die Stärke von Oberleder und Futter addiert, der fertige Therapie-Schuh derart dick, daß er nur noch Zusammen mit ebenfalls handgefertigten orthopädischen Spezialschuhen getragen werden kann. Solche Schuhe zeichnen sich jedoch nicht gerade durch besondere Eleganz aus und werden deshalb von vielen, insbesondere weiblichen, Patienten grundsätzlich abgelehnt.

Der vorliegenden Erfindung liegt die Aufgabe zugrunde, orthopädische Innenschuhe der eingangs genannten Art anzugeben, die eine optimale Fixierung des kranken Fußes verbinden mit einem minimalen Fertigungsaufwand dank industrieller Vorfertigung.

Diese Aufgabe wird gelöst durch Innenschuhe mit den Merkmalen des Anspruchs 1.

Damit ergeben sich die Vorteile, daß dank der Herstellung im Spritzgußverfahren Materialverteilung und -dicke der Rohlinge bereits der optimalen Endform entsprechen, daß die individuelle Anpassung an den Patienten-Leisten bzw. -Gipsabguß in einfachster Weise auch von Hilfspersonal optimal durchgeführt werden kann, daß Nachschleifen, Nachschmirgeln oder Beschneiden nicht mehr bzw. nur noch ausnahmsweise erforderlich sind, daß dank der vorgefertigten Taschen im Schuh- bzw. Innenschuhschaft die Arthrodesen-, Knöchel- bzw. Lähmungskappe in kürzester Zeit paßgenau mit dem Schaft verbunden werden kann, wobei auf die Verwendung von Klebstoff völlig verzichtet werden kann, wodurch es möglich wird, das Kunststoffmaterial ausgedienter Therapie-Schuhe bzw. -Innenschuhe zu recyclen, daß dank der einstückigen Herstellung von Kappe und Fußbett der Fuß sicher in der gewünschten optimalen Position zum Unterschenkel gehalten wird und daß dank der Möglichkeit, das Schnürteil als letzten Arbeitsgang mit dem aufgeleisteten, komplettierten Schuhschaft zu verbinden, der fertige Therapie-Schuh exakt den Beinmaßen des Patienten entspricht. Dank der Tatsache, daß das Kunststoffmaterial beim Auftritt und an den übergangsstellen zum Bein bzw. Fuß des Patienten geschärft und an der Ferse offen ist, kann der Therapie-Schuh als Innenschuh oder Manschette auch in modischen Konfektionsschuhen getragen werden, wodurch die Akzeptanz bei den Patienten drastisch erhöht wird.

Einer weiteren Erhöhung der Akzeptanz dient die Tatsache, daß dank der Vorfertigung insbesondere des Innen-Schuhschaftes und des Schnürteiles modische Aspekte problemlos berücksichtigt werden können. Der Patient kann sich anhand eines Kataloges sein Wunschmodell schon vorab aussuchen.

Da das Kunststoffmaterial im Bereich der Knöchel und unter der Ferse sehr dünn gehalten ist, kann es auch für extreme Knöchelformen umgeformt werden. Auf Anweisung des Arztes kann die Knöchelpartie auch ganz ausgeschnitten werden.

Vorteilhafterweise ist das Kunststoffmaterial transparent. Dadurch können empfindliche Stellen, beispielsweise Wunden, Vernarbungen, Operationsstellen usw., durch Ausschnitte exakt freigelegt werden.

Gemäß einer vorteilhaften Weiterbildung der Erfindung weisen die Kunststoff-Kappen Perforationslöcher auf. Dies verbessert die Luftzirkulation, ohne die Festigkeit zu beeinträchtigen. Dank der Herstellung der Kappen im Soritzgußverfahren können diese Luftlöcher ohne Zusatzaufwand angebracht werden.

Da die Kunststoff-Kappe im Fersen- und Achillessehnenbereich ausgespart ist, werden Reib- und Druckeffekte vermieden. Außerdem bleibt auch bei angelegtem Innenschuh die Schuhgröße für Konfektionsschuhe unverändert. Vorzugsweise sind an der Kunststoff-Kappe Schnittlinien angeformt, die es dem Orthopädie-Schuhmacher ermöglichen, aus der industriell vorgefertigten Kunststoff-Kappe je nach Bedarf eine Lähmungskappe mit Beinklammer, eine hohe Lähmungskappe, eine mittelhohe Lähmungskappe, eine niedrige Lähmungskappe oder auch eine Knöchelkappe herzustellen. Er muß dazu lediglich an der entsprechenden Schnittlinie entlangschneiden.

Zur Fertigstellung des orthopädischen Innenschuhs kann gemäß einer bevorzugten Weiterbildung der Erfindung ein vorzugsweise hoher Innen-Schuhschaft vorgesehen sein, in dem Taschen vorgefertigt sind, die die leistengetreu verformte Kunststoff-Kappe und das Fußbett paßgenau aufnehmen, wobei ein Schnürteil auf den komplettierten, ausgeleisteten Schaft aufgesetzt, vorzugsweise geklebt ist.

Es versteht sich, daß auch Verschlußriemen mit Klettverschluß vorgesehen werden können.

Anhand der Zeichnung soll die Erfindung in Form von Ausführungsbeispielen näher erläutert werden. Es zeigen jeweils in schematischer, perspektivischer Darstellung
- Fig. 1: eine thermoplastische, spritzgegossene Kunststoff-Kappe zur Herstellung von Arthrodesen-Kappen sowie mit eingeformten Schnittlinien zur Herstellung von Lähmungs- bzw. Knöchelkappen,
- Fig. 2: eine Arthrodesen-Kappe mit angeformtem Fußbett,
- Fig. 3: einen vorgefertigten Innen-Schuhschaft,
- Fig. 4: eine einfache Arthrodesenlasche,
- Fig. 5: ein vorgefertigtes Schnürteil,
- Fig. 6: einen fertiggestellten Arthrodesen-Innenschuh am Patientenfuß und
- Fig. 7: einen fertiggestellten Lähmungsinnenschuh.

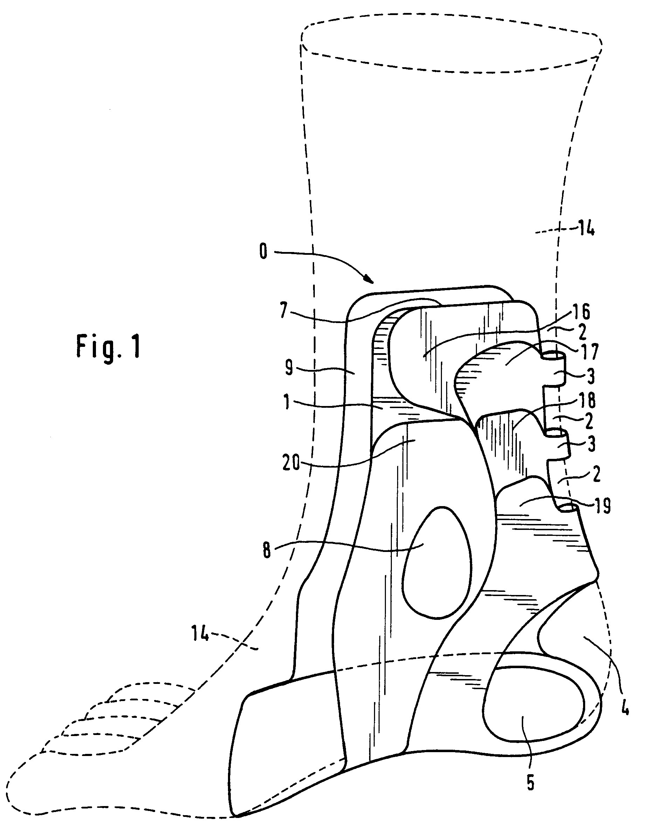

Fig. 1 zeigt eine Kunststoff-Kappe 0, welche zusammen mit einem Fußbett 5 einstückig aus thermoplastischem Kunststoff gespritzt ist. Der Achillessehnenbereich 2 ist ausgespart, um Reib- und Druckeffekte zu verringern. Damit die beiden Kappenteile nicht verrutschen und damit die vorgegebene Achillessehnenöffnung 2 formgetreu bleibt, sind Verbindungsstege 3 angeformt.

Auch ein Fersenbereich 4 ist ausgespart. Dadurch bleibt die Schuhkonfektionsgröße des Patienten auch beim Tragen des Therapie-Innenschuhs unverändert.

Aufgrund der Herstellung von Kunststoff-Kappe 0 und Fußbett 5 im Spritzgußverfahren ist es möglich, die Materialverteilung von vornherein gleich optimal zu gestalten, d. h. an den belasteten Stellen stärker, an den unbelasteten und empfindlichen Stellen dagegen schwächer. Eine Anpassung an die individuelle Form des Patientenfußes bzw. Patientenleistens 14 laßt sich ohne Probleme auch von Hilfspersonal vornehmen, indem das thermoplastische Kunststoffmaterial erwärmt und auf den Beinleisten 14 bzw. den Gipsabguß gepreßt wird. Ein Nachschleifen, Nachschmirgeln oder Beschneiden der Kunststoff-Kappe 0 ist für Arthrodesen-Kappen nicht, für Knöchel- bzw. Lähmungskappen kaum noch erforderlich.

Im Bereich der Knöchelpartie 8 ist das Kunststoffmaterial sehr dünn gehalten. Dadurch kann es auch für extreme Knöchelformen umgeformt werden. Auf Anweisung des Arztes kann man die Knöchelpartie 8 ganz ausschneiden.

Alle Ränder 7 im Bereich der Kunststoff-Kappe 0 und des Fußbettes 5, dort insbesondere im Bereich des Auftritts und der Ferse, sind geschärft, d. h. laufen dünn aus, wodurch sich weiche, elastische und somit für den Patienten angenehme Kantenübergänge zum Bein und Fuß ergeben. Gleichzeitig besitzt jedoch die spritzgegossene Kunststoff-Kappe 0 dank dieser Herestellungsart aine berechenbare und gleichbleibende Festigkeitsstruktur.

Es versteht sich, daß Polsterungen, Pelotten oder sonstige, die Versorgung des Patientenfußes bewirkende Maßnahmen ohne weiteres individuell angebracht werden können.

In die Kunststoff-Kappe 0 sind vergleichbar einem Schnittmuster Schnittlinien eingeformt, um aus der großen Kunststoff-Kappe 1, die für einen Arthrodesen-Schuh bzw. -Innenschuh bestimmt ist, eine Lähmungskappe 16 mit Beinklammer und Fußbett 5, eine hohe Lähmungskappe 17 mit Fußbett 5, eine mittelhohe Lähmungskappe 18 mit Fußbett 5, eine niedrige Lähmungskappe 19 mit Fußbett 5 oder auch eine sogenannte Knöchelkappe 20 mit Fußbett 5 herstellen zu können. Diese Methode hat den Vorteil, daß nicht für jeden Kappentyp und für jede Fußgröße eine eigene Spritzgußform hergestellt werden muß. Auch hält sich das Vorratslager beim Orthopädieschuhmacher so in Grenzen.

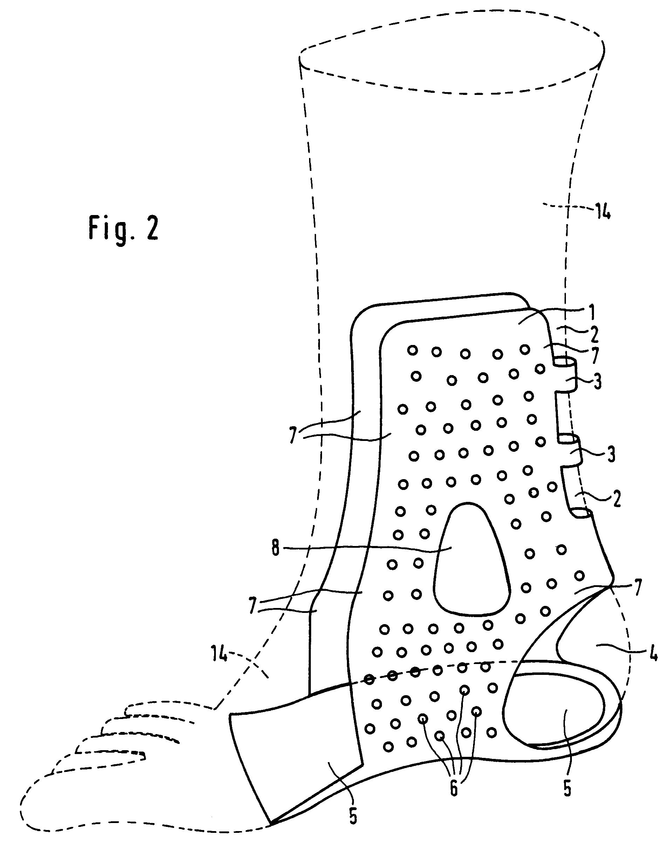

Fig. 2 zeigt eine unter Verwendung der Kunststoff-Kappe 0 der Fig. 1 hergestellte Arthrodesen-Kappe 1. Diese ist mit einer Vielzahl von Luftlöchern 6 versehen. Die Luftlöcher können dank der Herstellung der Kappe 1 im Spritzgußverfahren ohne Zusatzaufwand angebracht werden; sie verbessern die Luftzirkulation und damit die Trageigenschaften.

Eine materialabnehmende Nachbearbeitung ist bei dieser Arthrodesen-Kappe 1 nicht, allenfalls nur in besonderen Ausnahmefällen erforderlich.

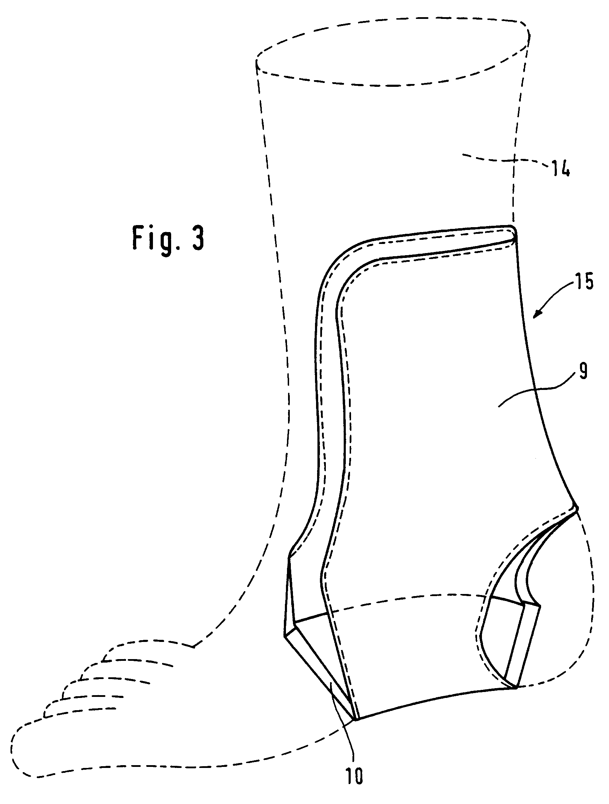

Fig. 3 zeigt einen teilweise vorgefertigten Innen-Schuhschaft 15 zur Herstellung eines Arthrodesen-Innenschuhs unter Verwendung der Arthrodesen-Kappe 1 gemäß Fig. 2. Dank der Tatsache, daß die Arthrodesen-Kappen 1 in jeder Schuhgröße mit jeweils gleicher Umfassungsgröße gefertigt werden, können auch die Schäfte 15 mit entsprechend paßgenauen Taschen 8 zwischen Oberleder und Futter vorgefertigt werden. Der Arthrodesen-Kern 1 wird, gegebenenfalls nach seiner individuellen Umformung gemäß dem Gipsabdruck oder Leisten 14, durch die Öffnung 10 in die Tasche 9 eingeschoben. Arthrodesen-Kappe 1 und Fußbett 5 sitzen fest in den vorgefertigten Taschen 9. Dadurch kann auf die Verwendung von Klebstoff verzichtet werden. Dies ermöglicht wiederum das Recycling des Kunststoffs aus ausgedienten Therapie-Schuhen.

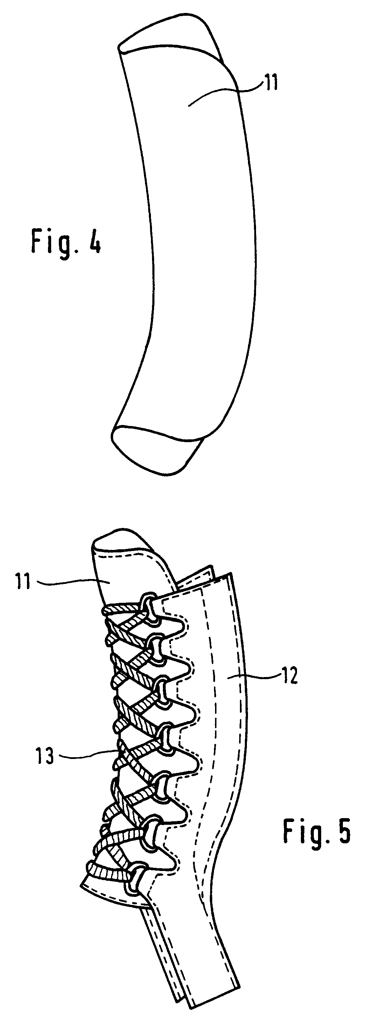

Fig. 4 zeigt eine einfache Arthrodesen-Lasche 11, wie sie aus Therapiegründen oftmals verwendet werden muß. Sie besteht ebenfalls aus thermoplastischem Kunststoff und wird mit denselben Verfahren hergestellt und endgeformt wie die Kunststoff-Kappe 0.

Fig. 5 zeigt ein vorgefertigtes Schnürteil 12. Dank der industriellen Vorfertigung können bei der Gestaltung der Zunge 11 bzw. der Schnürung 13, bei der Auswahl des Leders, der Lederfarbe usw. modische Aspekte berücksichtigt werden. Beispielsweise kann ein Modelleur mit der Gestaltung der Modelle beauftragt werden, da sich dessen Kosten auf die Serie umlegen lassen. Dank der weitgehenden Vorfertigung und dank der Möglichkeit, auf das Abschmirgeln von Kunststoff völlig zu verzichten, ergibt sich insgesamt eine derartige Material- und Zeitersparnis, daß Preiseinsparungen, aber auch eine beschleunigte Lieferung und damit oft eine Verkürzung der Krankenhausverweildauer erreicht werden können.

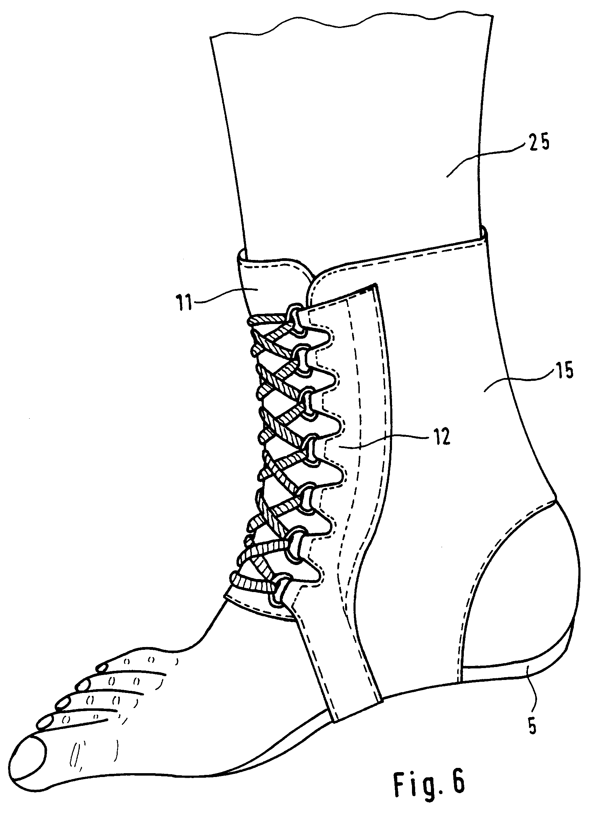

Fig. 6 schließlich zeigt das Aussehen des fertiggestellten Arthrodesen-Innenschuhs am Patientenfuß 25. Man erkennt, daß die Länge des Fußes durch den Innenschuh nicht, Höhe und Breite nur wenig verändert werden. Dadurch ist es möglich, auch bei angelegtem Arthrodesen-Innenschuh mit optimaler Stützfunktion Konfektionsschuhe zu tragen. Der Patient unterscheidet sich somit nicht mehr von einem Gesunden, insbesondere dann, wenn der hohe Schuhschaft 15 des Therapie-Schuhes beispielsweise unter einer langen Hose verschwindet.

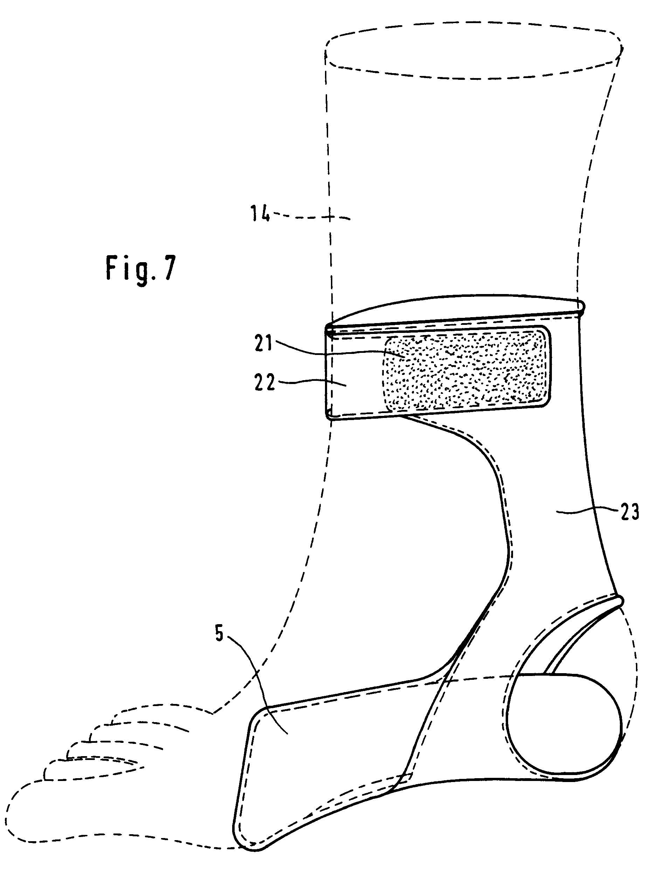

Fig. 7 zeigt einen Innenschuh mit einer hohen Lähmungskappe 16 mit Beinklammer und Fußbett 5, eingesetzt in eine industriell vorgefertigte Lähmungskappentasche 23. Zur Befestigung am Patientenbein 25, hier repräsentiert durch den Beinleisten 14, dient ein Verschlußriemen 22 mit Klettverschluß 21. Herstellung, Verarbeitung und Anwendung des Lähmungsinnenschuhs erfolgen analog zum Arthrodesen-Innenschuh der Fig. 6.

## Patentansprüche

1. Orthopädischer Innenschuh, umfassend eine beingerecht vorgeformte Kunststoff-Kappe (0) mit einstückig angeformtem Fußbett (5) und einer Aussparung (4) im Fersenbereich, wobei Kappe (0) und Fußbett (5) aus thermoplastischem Kunststoff bestehen, dadurch gekennzeichnet, daß die Kunststoff-Kappe (0) mit geschärften Rändern (7) formgespritzt ist, daß das Kunststoffmaterial im Bereich (8) der Knöchel und unter der Ferse sehr dünn gehalten ist, daß die Kunststoff-Kappe (0) auch im Achillessehnenbereich (2) derart ausgespart ist, daß Verbindungsstege (3) verbleiben, und daß die Kunststoff-Kappe (0) in Form und Größe einer Arthrodesen-Kappe (1) entspricht.

2. Innenschuh nach Anspruch 1, dadurch gekennzeichnet, daß das Kunststoffmaterial transparent ist.

3. Innenschuh nach Anspruch 1 oder 2, dadurch gekennzeichnet, daß die Kunststoff-Kappe (0) Perforationslöcher (6) aufweist.

4. Innenschuh nach Anspruch 1, 2 oder 3, dadurch gekennzeichnet, daß an der Kunststoff-Kappe (0) Schnittlinien angeformt sind zur Herstellung einer Lähmungskappe (16) mit Beinklammer, einer hohen Lähmungskappe (17), einer mittelhohen Lähmungskappe (18), einer niedrigen Lähmungskappe (19) bzw. einer Knöchelkappe (20).

5. Innenschuh nach einem der Ansprüche 1 bis 4, dadurch gekennzeichnet, daß ein vorzugsweise hoher Innen-Schuhschaft (15) vorgesehen ist, daß im Schuh- bzw. Innenschaft (15, 23) zwischen Oberleder und Futter Taschen (9) vorgefertigt sind, die die leistengetreu verformte Kunststoff-Kappe (0) und das Fußbett (5) paßgenau aufnehmen, und daß ein Schnürteil (12; 21, 22) auf den komplettierten, ausgeleisteten Schuh- bzw. Innenschaft aufgesetzt, vorzugsweise geklebt ist.

6. Innenschuh nach Anspruch 5, dadurch gekennzeichnet, daß ein Verschlußriemen (22) mit Klettverschluß (21) vorgesehen ist.

## Claims

1. An orthopaedic inner shoe, comprising a preformed plastics cap (0) adapted to the leg with an integrally formed orthopaedic insole (5) and an opening (4) in the heel region, wherein the cap (0) and the orthopaedic insole (5) consist of thermoplastic synthetic material, characterized in that the plastics cap (0) is injection-moulded with tapered edges (7), in that the plastics material is kept very thin in the region (8) of the ankle and under the heel, in that the plastics cap (0) is also cut away in the Achilles tendon region (2) so as to leave connecting webs (3), and in that the plastics cap (0) corresponds in shape and size to an arthrodesis cap (1).

2. An inner shoe according to Claim 1, characterized in that the plastics material is transparent.

3. An inner shoe according to Claim 1 or 2, characterized in that the plastics cap (0) has perforation holes (6).

4. An inner shoe according to Claim 1, 2 or 3, characterized in that cutting lines are formed in the plastics cap (0) with a view to producing a paralysis cap (16) with a leg clamp, an upper paralysis cap (17), a middle paralysis cap (18), a lower paralysis cap (19) and/or an ankle cap (20).

5. An inner shoe according to any one of Claims 1 to 4, characterized in that a preferably upper inner-shoe leg (15) is provided, in that pockets (9) are prefabricated in the shoe leg and inner leg (15 and 23 respectively) between the upper and lining, which pockets (9) accommodate in an accurately fitting manner the plastics cap (0), which has been accurately shaped on the last, and the orthopaedic insole (5), and in that a laced part (12; 21, 22) is fitted, preferably bonded, to the completed shoe leg and inner leg removed from the last.

6. An inner shoe according to Claim 5, characterised in that a fastening strap (22) with a hook and loop-type fastener (21) is provided.

## Revendications

1. Chausson orthopédique, comprenant un capuchon en matière synthétique (O) préformé à la forme de la jambe, avec une base de pied (5) formée d'un seul tenant et un évidement (4) dans la zone du talon, le capuchon (O) et la base de pied (5) étant constitués d'une matière synthétique thermoplastique, caractérisé en ce que le capuchon en matière synthétique (O) est moulé par injection, avec des bords (7) vifs, en ce que le matériau synthétique dans la zone (8) des malléoles et sous le talon est laissé très mince, en ce que le capuchon en matière synthétique (O) est également évidé dans la zone du tendon d'Achille (2), de manière que subsistent des bandes de liaison (3) et en ce que le capuchon en matière synthétique (O) corresponde en forme et en taille à un capuchon d'arthrodèse (1).

2. Chausson selon la revendication 1, caractérisé en ce que le matériau synthétique est transparent (3).

3. Chausson selon la revendication 1 ou 2, caractérisé en ce que le capuchon en matière synthétique (O) présente des trous d'aération (6).

4. Chausson selon la revendication 1, 2 ou 3, caractérisé en ce que sur le capuchon de matière synthétique (O) sont ménagées des lignes de coupe, pour constituer un capuchon paralysant (16) avec une pince à jambe, un capuchon paralysant haut (17), un capuchon paralysant de hauteur moyenne (18), un capuchon paralysant bas (19), respectivement un capuchon à malléoles (20).

5. Chausson selon l'une des revendications 1 à 4, caractérisé en ce qu'est prévu une tige de chaussure intérieure (15) de préférence haute, en ce que, dans la tige de chaussure respectivement intérieur (15, 23) sont préfabriquées, entre le cuir de dessus et la garniture, des poches (9) qui reçoivent avec un ajustement précis le capuchon en matière synthétique (O) ayant la forme de l'embauchoir et la base de pied (5) et en ce qu'une partie de laçage (12; 21, 22) est appliquée sur la tige de chaussure respectivement intérieure complétée, mise à la forme de l'embauchoir, l'application se faisant de préférence par collage.

6. Chausson selon la revendication 5, caractérisé en ce qu'est prévue une sangle de fermeture (22) avec une fermeture auto-accrochante (21).
